# EUROPÄISCHE PATENTSCHRIFT

(11) **EP 0 791 032 B1**
(45) Veröffentlichungstag und Bekanntmachung des Hinweises auf die Patenterteilung: **03.05.2000**
(21) Anmeldenummer: 95938396.9
(22) Anmeldetag: 03.11.1995
(51) Int. Cl.: C08K 5/10, C08L 101/00

(54) **VERWENDUNG VON 3-ARYLACRYLSÄUREESTERN ALS LICHTSCHUTZMITTEL UND STABILISATOREN FÜR NICHT LEBENDES ORGANISCHES MATERIAL**
USE OF 3-ARYLACRYLIC ACID ESTERS AS LIGHT-PROTECTION AGENTS AND STABILIZERS FOR NON-LIVING ORGANIC MATERIAL
UTILISATION D'ESTERS DE L'ACIDE 3-ARYLACRYLIQUE COMME AGENTS DE PROTECTION CONTRE LA LUMIERE ET COMME STABILISANTS POUR UN MATERIAU ORGANIQUE NON VIVANT

(30) Priorität: 11.11.1994 DE 4440288
(43) Veröffentlichungstag der Anmeldung: 27.08.1997
(73) Patentinhaber: BASF AKTIENGESELLSCHAFT, 67056 Ludwigshafen (DE)
(72) Erfinder: AUMÜLLER, Alexander, D-67435 Neustadt (DE); HOLDERBAUM, Martin, D-67065 Ludwigshafen (DE); GOETZE, Wolfgang, D-67133 Maxdorf (DE); KROCKENBERGER, Jürgen, D-67063 Ludwigshafen (DE); TRAUTH, Hubert, D-67373 Dudenhofen (DE)
(86) Internationale Anmeldenummer: EP9504313
(87) Internationale Veröffentlichungsnummer: WO9615184

(56) Entgegenhaltungen:
- DE-A- 1 922 815
- DE-A- 3 012 535
- US-A- 4 049 869
- US-A- 4 273 833
- DATABASE WPI Section Ch, Week 7841 Derwent Publications Ltd., London, GB; Class A60, AN 78-73767a & JP,A,53 102 357 (ADEKA ARGUS) , 6.September 1978
- DATABASE WPI Section Ch, Week 9336 Derwent Publications Ltd., London, GB; Class A60, AN 93-285385 & JP,A,05 201 928 (CHIKUNO SHOKUHIN KKK) , 10.August 1993
- DATABASE WPI Section Ch, Week 9540 Derwent Publications Ltd., London, GB; Class A17, AN 95-309225 & JP,A,07 207 063 (TOPPAN PRINTING) , 8.August 1995

## Beschreibung

Die vorliegende Erfindung betrifft die Verwendung von 3-Arylacrylsäureestern der allgemeinen Formel I 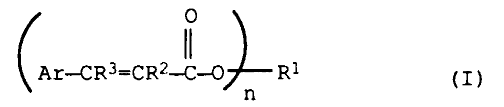 in der
- Ar: für einen Arylrest steht, welcher zusätzlich Substituenten tragen kann,
- R¹: den Rest eines n-wertigen aliphatischen Polyols mit bis zu 20 C-Atomen, wobei die Kohlenstoffkette durch bis zu 9 nicht benachbarte Sauerstoffatome unterbrochen sein kann, oder eines n-wertigen cycloaliphatischen Polyols mit 5 bis 20 C-Atomen, in welchen Ringkohlenstoffatome auch durch nichtbenachbarte Sauerstoffatome ersetzt sein können, bezeichnet,
- R² und R³: Wasserstoff oder C₁- bis C₄-Alkyl bedeuten und
- n: für eine Zahl von 1 bis 10 steht,
als Lichtschutzmittel und Stabilisatoren für Polyurethane und Polycarbonate.

3-Arylacrylsäureester des obengenannten Typs, beispielsweise p-Methoxyzimtsäure-2-ethylhexylester, sind als Lichtschutzmittel in der Kosmetik, d.h. zum Schutz der menschlichen Haut, seit langem bekannt.

Zum Schutz von nicht lebendem organischem Material, insbesondere von Kunststoffen und Lacken, sind bislang Verbindungen anderer chemischer Strukturen eingesetzt worden, beispielsweise sterisch gehinderte Amine des Polyalkylpiperidin-Typs oder Benztriazol-Derivate. Solche Mittel sollen der Zerstörung von organischem Material entgegenwirken, die sich üblicherweise in einer Vergilbung oder Verfärbung sowie in einer Versprödung des organischen Materials zeigt.

Unbefriedigend ist bei den genannten Mitteln des Standes der Technik häufig noch die zu geringe Verträglichkeit mit Kunststoffen, die zu geringe Dauer der Schutzwirkung, die Eigenfarbe der Substanzen, die Neigung zur Flüchtigkeit und die thermische Zersetzung der Stabilisatoren beim Einarbeiten bei erhöhter Temperatur.

Aufgabe der vorliegenden Erfindung war es daher, Lichtschutzmittel bzw. Stabilisatoren bereitzustellen, die einen wirkungsvolleren Schutz für organisches Material mit sich bringen. Insbesondere sollte die Dauer der Schutzwirkung verlängert werden.

Demgemäß wurde die Verwendung der eingangs definierten 3-Arylacrylsäureester I gefunden.

In einer bevorzugten Ausführungsform steht Ar für einen Phenyl-, Biphenyl- oder Napthylrest, der durch ein bis drei C₁-C₄-Alkylgruppen, C₁-C₄-Alkoxygruppen, Hydroxylgruppen, Phenoxygruppen, Aminogruppen, welche durch C₁-C₄-Alkylgruppen mono- oder disubstituiert sein können, Halogenatome, Nitrogruppen oder eine Methylendioxygruppe substituiert sein kann, wobei die Substituenten gleich oder verschieden sein können.

Als Beispiele für Ar sind zu nennen:
Phenyl,
o-, m- oder p-Tolyl,
o-, m- oder p-Ethylphenyl,
o-, m- oder p-Propylphenyl,
m- oder p-Cumyl,
o-, m- oder p-Butylphenyl,
m- oder p-iso-Butylphenyl,
m- oder p-sec.-Butylphenyl,
m- oder p-tert.-Butylphenyl,
2,3-, 2,4-, 2,5-, 2,6-, 3,4- oder 3,5-Dimethylphenyl,
Mesityl,
o-, m- oder p-Methoxyphenyl,
o-, m- oder p-Ethoxyphenyl,
o-, m- oder p-Propoxyphenyl,
m- oder p-iso-Propoxyphenyl,
o-, m- oder p-Butoxyphenyl,
m- oder p-iso-Butoxyphenyl,
m- oder p-sec.-Butoxyphenyl,
m- oder p-tert.-Butoxyphenyl,
2,3-, 2,4-, 2,5-, 2,6-, 3,4- oder 3,5-Dimethoxyphenyl,
o-, m- oder p-Hydroxyphenyl,
2,3-, 2,4-, 2,5-, 2,6-, 3,4- oder 3,5-Dihydroxyphenyl,
3-Hydroxy-4-methoxyphenyl,
m- oder p-Phenoxyphenyl,
o-, m- oder p-Aminophenyl,
o-, m- oder p-(N-Methylamino)phenyl,
o-, m- oder p-(N,N-Dimethylamino)phenyl,
o-, m- oder p-Fluorphenyl,
o-, m- oder p-Chlorphenyl,
2,4-Dichlorphenyl,
o-, m- oder p-Bromphenyl,
o-, m- oder p-Nitrophenyl,
2,3- oder 3,4-Methylendioxyphenyl,
2-, 3- oder 4-Biphenyl und
α- oder β-Naphthyl.

Besonders bevorzugt werden C₁-C₄-Alkoxyphenylreste, insbesondere, wenn der Alkoxyrest in p-Position am Phenylkern steht. Unter diesen Resten ist der p-Methoxyphenylrest besonders hervorzuheben.

Im Fall n = 1 sind Beispiele für R¹ die folgenden aliphatischen Reste: Methyl, Ethyl, n-Propyl, iso-Propyl, n-Butyl, iso-Butyl, sek.-Butyl, tert.-Butyl, n-Pentyl, iso-Pentyl, sek.-Pentyl, tert.-Pentyl, neo-Pentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Hexadecyl, n-Octadecyl, n-Eicosyl, Vinyl, Allyl, Methallyl, Oleyl, Linolyl und Linolenyl. Hiervon werden geradkettige oder verzweigte C₅- bis C₁₆-Alkylgruppen, insbesondere geradkettige oder verzweigte C₈- bis C₁₂-Alkylgruppen, bevorzugt. Von besonderem Interesse sind geradkettige oder verzweigte C₈-Alkylgruppen, darunter besonders der 2-Ethylhexylrest. Als cycloaliphatische Reste kommen beispielsweise Cyclopentyl, Cyclohexyl, Cycloheptyl und Cyclooctyl in Betracht, wobei diese Gruppen auch mit Alkylgruppen ein- oder mehrfach substituiert sein können.

Im Falle n = 2 bis 10 kommen als Beispiele für von Polyolen der Formel R¹(OH)ₙ abgeleitete n-wertige Reste R¹ insbesondere solche mit 2 bis 12 C-Atomen, welche in ihrem linearen oder verzweigten Kohlenstoffgerüst durch bis zu 3 nicht benachbarte Sauerstoffatome unterbrochen sein können, oder welche in ihrem cyclischen Kohlenstoffgerüst einzelne Sauerstoffatome enthalten können, in Betracht. Einzelne Beispiele hierfür sind:

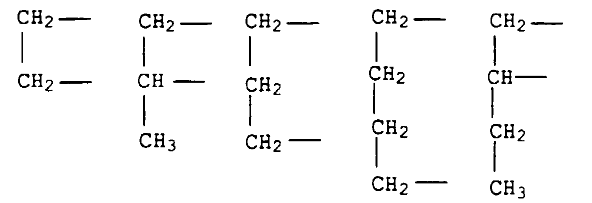

―CH₂CH₂―O―CH₂CH₂― ―CH₂CH₂―O―CH₂CH₂―O―CH₂CH₂―

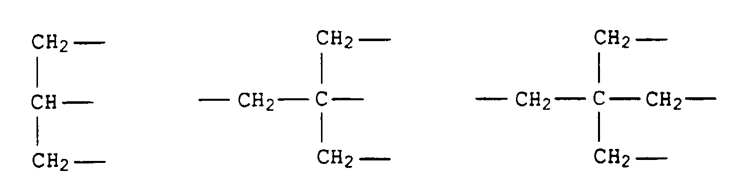

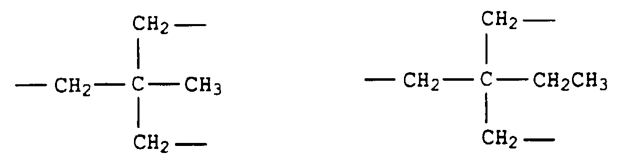

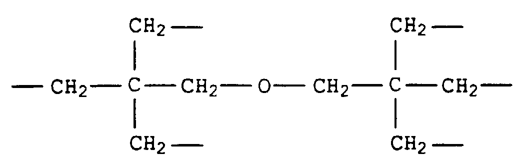

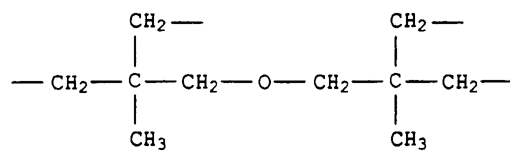

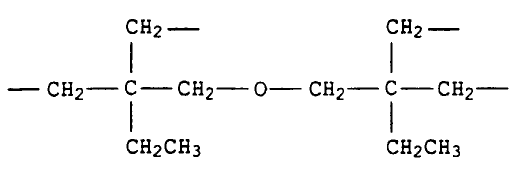

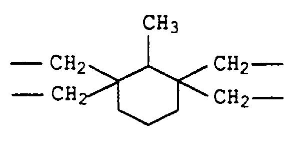

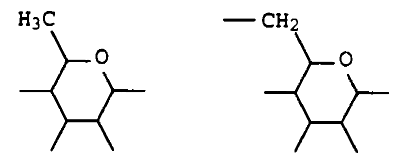

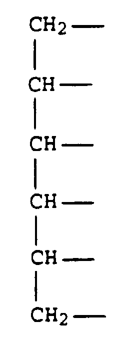

Als mehrwertige cycloaliphatische Reste kommen z.B. solche in Betracht, die sich von den mehrwertigen Alkoholen 1,3-Cyclopentandiol, 1,3-Cyclohexandiol oder besonders vom 1,4-Cyclohexandiol oder 1,4-Cyclohexandimethanol ableiten, wobei die Cycloalkylringe auch durch weitere Alkylgruppen substituiert sein können.

Die Variable n steht vorzugsweise für eine Zahl von 1 bis 6, insbesondere von 1 bis 4, vor allem jedoch für die Zahl 1 oder 2.

Die Reste R² und R³ bedeuten unabhängig voneinander insbesondere Wasserstoff, Methyl oder Ethyl. Ganz besonders bevorzugt werden Verbindungen I, bei denen R² Wasserstoff oder Methyl und R³ Wasserstoff bedeutet.

Die 3-Arylacrylsäureester I eignen sich in hervorragender Weise zum Stabilisieren von Polyurethanen und Polycarbonaten gegen die Einwirkung von Licht, Sauerstoff und Wärme. Sie sind auch wirksam als Metalldesaktivatoren. Sie werden dem zu stabilisierenden Polyurethan oder Polycarbonat in einer Konzentration von 0,01 bis 5 Gew.-%, vorzugsweise von 0,02 bis 2 Gew.-%, bezogen auf das Polyurethan oder Polycarbonat, vor, während oder nach seiner Herstellung zugesetzt.

Gegenstand der vorliegenden Erfindung ist außerdem gegen die Einwirkung von Licht, Sauerstoff und Wärme stabilisiertes Polyurethan oder Polycarbonat, welches eine oder mehrere der Verbindungen I in den oben angegebenen Konzentrationen enthält.

Zur Vermischung der Verbindungen I mit Polyurethanen oder Polycarbonaten können alle bekannten Vorrichtungen und Methoden zum Einmischen von Stabilisierungsmitteln oder anderen Zusätzen in Polymere angewandt werden.

Die durch die Verbindungen I stabilisierten Polyurethane oder Polycarbonate können gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den Verbindungen I zugesetzt werden können, sind z.B. Verbindungen auf der Basis sterisch gehinderter Phenole sterisch gehinderter Amine, Chromanderivate oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxybenzyl)-propionylethyl]-iso-cyanurat, 1,3,5-Tris-(2,6-dimethyl-3-hydroxy-4-tert.-butylbenzyl)-isocyanurat und Pentaerythrit-tetrakis-[β-3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Distearylpentaerythritdiphosphit, Tris-(2-tert.-butyl-4-methylphenyl)-phosphit, Bis-(2,4-di-tert.-butyl-phenyl)-pentaerythritdiphosphit und Tetrakis- (2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-β-laurylthiopropionat) und Pentaerythrittetrakis-(-β-hexylthiopropionat) genannt. Weiterhin können Thiobisphenole wie 3,3'-Di-tert.-butyl-4,4'-dihydroxy-2,2'-dimethyl-diphenylsulfid zugesetzt werden.

Weitere Antioxidantien und Lichtstabilisatoren, die zusammen mit den Verbindungen I verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den Verbindungen I noch mindestens ein weiterer Lichtstabilisator aus der Verbindungsklasse der sterisch gehinderten Amine in üblicher Konzentration zugesetzt wird.

Als weitere sterisch gehinderte Amine kommen hierfür z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin mit Bernsteinsäure, das Kondensationsprodukt von N,N'-(2,2,6,6-tetramethylpiperidyl)-hexamethylendiamin mit 4-tert.-Octylamino-2,6-dichlor-1,3,5-triazin, Tris-(2,2,6,6-tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen mit Tetramethylolacetylendiharnstoffen.

Eine besonders gute Stabilisierung für Polyurethane erhält man, wenn man zu den Verbindungen I noch ein sterisch gehindertes Amin, z.B. Bis-(2,2,6,6-tetramethylpiperidyl)-sebacat oder Bis-(1,2,2,6,6-pentamethylpiperidyl)-sebacat, und zusätzlich ein Gemisch aus einem Chromanderivat (Vitamin E, α-Tocopherol), einem organischen Phosphit und einem Amin, wie es in der deutschen Patentanmeldung P 44 05 670.2 beschrieben ist, in üblicher Konzentration zusetzt.

Auch eine Mischung aus den Verbindungen I mit einem sterisch gehinderten Amin und einem Chromanderivat eignet sich besonders vorteilhaft zur Stabilisierung von Polyurethanen.

Bevorzugt werden die Verbindungen I zum Stabilisieren von Polyurethanen, insbesondere von Polyurethanschäumen, aber auch von Polyurethanformmassen, von Polycarbonaten und von Fahrzeuglackierungen, insbesondere auf dem Automobil-Sektor.

Die Verbindungen I zeichnen sich durch eine gute Verträglichkeit mit den üblichen Kunststoffarten und durch eine gute Löslichkeit und eine ausgezeichnete Verträglichkeit in den üblichen Lacksystemen aus. Sie sind bei den üblichen Kunststoff- und Lack-Verarbeitungstemperaturen stabil und nicht flüchtig. Als besondere Vorteile gegenüber bekannten Stabilisatoren seien hervorgehoben, daß die erfindungsgemäß zu verwendenden Verbindungen I eine deutlich geringere Eigenfarbe aufweisen und damit den neutralen Farbton insbesondere von transparenten Kunststoffen wie Polycarbonaten, Polyurethanschäumen und Lacken praktisch nicht beeinflussen. Darüber hinaus zeigen die erfindungsgemäß verwendeten Verbindungen I einen verbesserten Stabilisatoreffekt, d.h. die Schädigung des Materials setzt in ihrer Gegenwart wesentlich später ein.

### Anwendungsbeispiele

### Beispiel 1 (Polycarbonat)

0,20 Gew.-Teile der Verbindung p-Methoxyzimtsäure-2-ethylhexylester (A) wurden in 100 Gew.-Teilen Polycarbonat (Macrolon® PC 2800 der Fa. Bayer) durch einmaliges Extrudieren bei 280°C eingearbeitet und das anfallende Granulat über eine Spritzgußmaschine bei 300°C zu 2 mm dicken Prüfkörpern gespritzt.

Die so hergestellten Prüflinge wurden in einem Xenotest® 1200-Schnellbewitterungsgerät auf ihre Licht- und Wetterechtheit getestet. Die Alterung wurde durch Messen des Yellowness-Indexes nach ASTM D 1925 nach definierten Zeitintervallen bestimmt. Die Belichtungsdauer betrug bei allen Versuchen maximal 2000 Stunden.

Als Vergleich zum Stand der Technik dient das Benztriazol-Derivat der Formel B, das in gleichen Mengen in das gleiche Polycarbonat analog eingearbeitet und analog getestet wurde.

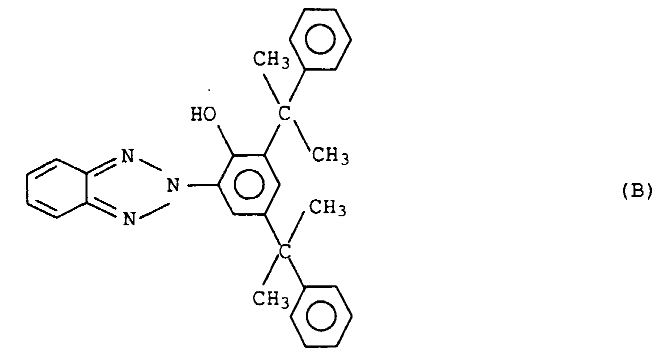

Die Ergebnisse sind in Tabelle 1 zusammengestellt.

**Tabelle 1**

| Stabilisator | Yellowness-Index nach Stunden (Xenotest 1200) | | | |
|---|---|---|---|---|
| | 0 | 1000 | 1500 | 2000 |
| ohne Stabilisator | 10,3 | 15,5 | 20,1 | 21,8 |
| A (erfindungsgemäß) | 10,2 | 11,0 | 14,2 | 15,3 |
| B (zum Vergleich) | 11,5 | 12,7 | 15,6 | 16,8 |

### Beispiel 2 (Polyurethanschaum)

### (Herstellung der Belichtungsproben):

Eine Polyolkomponente aus 41,9 g eines Polyetherols (OH-Zahl: 29,0), das durch Addition von Propylenoxid und Ethylenoxid an Propylenglykol erhalten wurde und ungefähr 84 Gew.-% primäre Hydroxylgruppen besaß, 42,5 g eines Polyetherols (OH-Zahl: 27,0), das durch Addition von Propylenoxid und Ethylenoxid an Trimethylolpropan erhalten wurde und ungefähr 88 Gew.-% primäre Hydroxylgruppen besaß, 8,1 g 1,4-Butandiol, 1,724 g einer 25 gew.-%igen Lösung von Diazabicyclooctan in 1,4-Butandiol, 0,016 g Dibutylzinndilaurat, 0,1 g des Siliconstabilisators OS 710 der Firma Bayer, 5,49 g Fluortrichlormethan und 0,17 ml Wasser wurde mit den im folgenden genannten Stabilisatoren (jeweils 0,5 g) versetzt und im Gewichtsverhältnis 100:48,5 mit einem Präpolymeren, das 23,0 Gew.-% Isocyanatgruppen aufwies, bei 25°C (Komponenten- und Werkzeugtemperatur) zu Prüfplatten verschäumt. Das NCO-Präpolymer wurde dabei hergestellt aus 87,17 g 4,4'-Diphenylmethandiisocyanat, 4,83 g eines Polyetherols (OH-Zahl: 250), das durch Addition von Propylenoxid an Propylenglykol erhalten wurde, und 8,0 g Dipropylenglykol.

Als erfindungsgemäß verwendete Verbindung diente die Verbindung A aus Beispiel 1. Als Vergleich zum Stand der Technik diente die aus den beiden angegebenen Komponenten erhaltene Verbindung C.

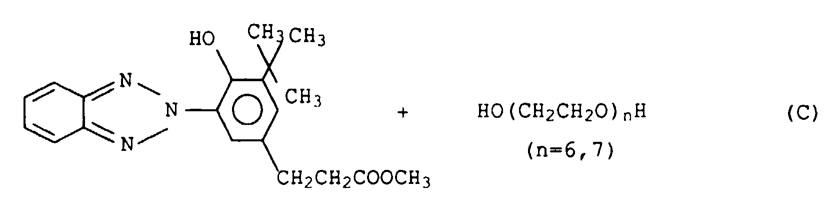

Die Prüfplatten wurden gemäß Xenotest 450 belichtet und danach der Yellowness Index gemäß ASTM D 1925 bestimmt. Die Ergebnisse sind in Tabelle 2 angegeben.

**Tabelle 2**

| Stabilisator | Yellowness-Index nach Stunden (Xenotest 450) | |
|---|---|---|
| | 0 | 240 |
| ohne Stabilisator | 2,8 | 45,2 |
| A (erfindungsgemäß) | 3,0 | 17,0 |
| C (zum Vergleich) | 3,4 | 18,4 |

## Patentansprüche

1. Verwendung von 3-Arylacrylsäureestern der allgemeinen Formel I 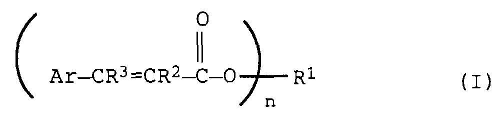 in der
Ar für einen Arylrest steht, welcher zusätzlich Substituenten tragen kann,
R¹ den Rest eines n-wertigen aliphatischen Polyols mit bis zu 20 C-Atomen, wobei die Kohlenstoffkette für n > 2 durch bis zu 9 nicht benachbarte Sauerstoffatome unterbrochen sein kann, oder eines n-wertigen cycloaliphatischen Polyols mit 5 bis 20 C-Atomen, in welchem Ringkohlenstoffatome auch durch nichtbenachbarte Sauerstoffatome ersetzt sein können, bezeichnet,
R² und R³ Wasserstoff oder C₁- bis C₄-Alkyl bedeuten und
n für eine Zahl von 1 bis 10 steht,
als Lichtschutzmittel und Stabilisatoren für Polyurethane und Polycarbonate.

2. Verwendung von 3-Arylacrylsäureestern I nach Anspruch 1, bei denen Ar für einen C₁- bis C₄-Alkoxyphenylrest steht.

3. Verwendung von 3-Arylacrylsäureestern I nach Anspruch 1 oder 2, bei denen Ar für p-Methoxyphenyl steht.

4. Verwendung von 3-Arylacrylsäureestern I nach den Ansprüchen 1 bis 3, bei denen R¹ den Rest eines geradkettigen oder verzweigten aliphatischen Polyols mit 5 bis 16 C-Atomen bezeichnet.

5. Verwendung von 3-Arylacrylsäureestern I nach den Ansprüchen 1 bis 4, bei denen R¹ für 2-Ethylhexyl steht.

6. Verwendung von 3-Arylacrylsäureestern I nach den Ansprüchen 1 bis 5, bei denen R² Wasserstoff oder Methyl und R³ Wasserstoff bedeutet.

7. Verwendung von 3-Arylacrylsäureestern I nach den Ansprüchen 1 bis 6, bei denen n für die Zahl 1 steht.

8. Gegen die Einwirkung von Licht, Sauerstoff und Wärme stabilisiertes Polyurethan oder Polycarbonat, enthaltend 0,01 bis 5 Gew.-%, bezogen auf die Menge des Polyurethans oder Polycarbonats, eines oder mehrerer der 3-Arylacrylsäureester I gemäß den Ansprüchen 1 bis 7.

## Claims

1. The use of 3-arylacrylic esters of the general formula I 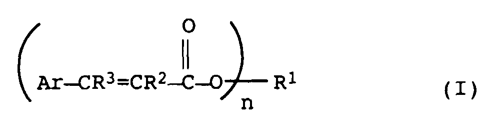 where
Ar is an aryl radical which can additionally carry substituents,
R¹ is the residue of an n-hydric aliphatic polyol having up to 20 carbon atoms, where the carbon chain for n > 2 can be interrupted by up to 9 non-adjacent oxygen atoms, or of an n-hydric cyclo-aliphatic polyol having 5 to 20 carbon atoms in which ring carbon atoms can also be replaced by non-adjacent oxygen atoms,
R² and R³ are hydrogen or C₁-C₄-alkyl, and
n is a number from 1 to 10,
as stabilizers, in particular against the action of light, for polyurethanes and polycarbonates.

2. The use of 3-arylacrylic esters I as claimed in claim 1, where Ar is a C₁-C₄-alkoxyphenyl radical.

3. The use of 3-arylacrylic esters I as claimed in claim 1 or 2, where Ar is p-methoxyphenyl.

4. The use of 3-arylacrylic esters I as claimed in any of claims 1 to 3, where R¹ is the residue of a straight-chain or branched aliphatic polyol having 5 to 16 carbon atoms.

5. The use of 3-arylacrylic esters I as claimed in any of claims 1 to 4, where R¹ is 2-ethylhexyl.

6. The use of 3-arylacrylic esters I as claimed in any of claims 1 to 5, where R² is hydrogen or methyl and R³ is hydrogen.

7. The use of 3-arylacrylic esters I as claimed in any of claims 1 to 6, where n is 1.

8. A polyurethane or polycarbonate which is stabilized against the action of light, oxygen and heat and contains from 0.01 to 5% by weight, based on the amount of the polyurethane or polycarbonate, of one or more 3-arylacrylic esters I as set forth in any of claims 1 to 7.

## Revendications

1. Utilisation d'esters d'acide 3-arylacrylique de formule générale I 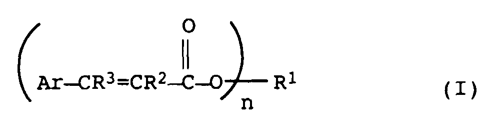 dans laquelle
Ar est mis pour un reste aryle pouvant porter en outre des substituants,
R¹ est mis pour le reste d'un polyol aliphatique n-valent ayant jusqu'à 20 atomes de C, la chaîne carbonée pouvant être interrompue par jusqu'à 9 atomes d'oxygène non voisins, lorsque n>2, ou d'un polyol cycloaliphatique n-valent ayant de 5 à 20 atomes de C, dans lequel les atomes de carbone du cycle peuvent également être remplacés par des atomes d'oxygène non voisins,
R² et R³ représentent des atomes d'hydrogène ou des groupements alkyle en C₁-C₄ et
n est mis pour un nombre de 1 à 10,
en tant qu'agent protecteur contre la lumière et qu'agent stabilisant pour des polyuréthanes et des polycarbonates.

2. Utilisation d'esters d'acide 3-arylacrylique I selon la revendication 1, pour lesquels Ar est mis pour un reste (alcoxy en C₁-C₄)phényle.

3. Utilisation d'esters d'acide 3-arylacrylique I selon la revendication 1 ou 2, pour lesquels Ar est mis pour un reste p-méthoxyphényle.

4. Utilisation d'esters d'acide 3-arylacrylique I selon l'une quelconque des revendications 1 à 3, pour lesquels R¹ représente le reste d'un polyol aliphatique linéaire ou ramifié ayant de 5 à 16 atomes de C.

5. Utilisation d'esters d'acide 3-arylacrylique I selon l'une quelconque des revendications 1 à 4, pour lesquels R¹ représente un reste 2-éthylhexyle.

6. Utilisation d'esters d'acide 3-arylacrylique I selon l'une quelconque des revendications 1 à 5, pour lesquels R² représente un atome d'hydrogène ou un groupement méthyle et R³ représente un atome d'hydrogène.

7. Utilisation d'esters d'acide 3-arylacrylique I selon l'une quelconque des revendications 1 à 6, pour lesquels n est mis pour le nombre 1.

8. Polyuréthane ou polycarbonate stabilisé contre l'action de la lumière, de l'oxygène et de la chaleur, contenant 0,01-5% en poids, par rapport à la quantité de polyuréthane ou de polycarbonate, d'un ou plusieurs esters d'acide 3-arylacrylique I selon l'une quelconque des revendications 1 à 7.
